# EUROPEAN PATENT SPECIFICATION

(11) **EP 1 933 709 B1**
(45) Date of publication and mention of the grant of the patent: **21.09.2011**
(21) Application number: 06794690.5
(22) Date of filing: 09.10.2006
(51) Int. Cl.: A61B 6/12, A61B 19/00, A61B 17/00, A61B 5/055

(54) **LOCATION AND STABILIZATION DEVICE**
LOKALISATIONS- UND STABILISATIONSVORRICHTUNG
DISPOSITIF DE LOCALISATION ET DE STABILISATION

(30) Priority: 11.10.2005 GB 0520596
(43) Date of publication of application: 25.06.2008
(73) Proprietor: Sussex Development Services LLP, Haywards Heath, West Sussex, RH16 1BP (GB); The Institute of Cancer Research: Royal Cancer Hospital, London SW7 3RP (GB)
(72) Inventor: BULBROOK, George, Lee, Norfolk NR20 4AJ (GB); DEARNALEY, David, Paul, Surrey SM5 3RA (GB)
(74) Representative: ip21 Ltd
(86) International application number: PCT/GB2006/003739
(87) International publication number: WO 2007/042777

(56) References cited:
- WO-A-2005/046792
- WO-A1-03/088833
- WO-A2-03/070294
- US-A- 5 404 881
- US-A- 5 797 950
- US-A1- 2002 143 275

## Description

### Field of the Invention

The invention relates to devices to assist a clinician in locating the position of an internal organ of a patient, especially the prostate gland, by use of non-invasive imaging.

### Background

Prostate cancer is the most common cancer of men in the developed world. Following diagnosis, several treatment options are available; one of these is radiotherapy. During treatment, the tumour must be exposed to radiation to treat the cancerous cells, and it is therefore important that an attending clinician can locate the position of the prostate. Accurate location of the prostate is important to reduce the risk of damage to adjacent tissues, such as the walls of the anus, rectum, and colon.

One technique that has been used to assist with this process is the insertion of a number of gold "seeds" into the prostate. The seeds - each about the size of a grain of rice - are put in place, usually with the help of ultrasound imaging, using long hollow needles through which the seeds are injected. The gold seeds are opaque to X-rays, and so may be used to "triangulate" the position of the prostate. The prostate is not usually visible on X-ray images, but computer tomography may be used after implantation of the seeds, to image both the seeds and the prostate, to determine the position of the seeds relative to the prostate gland. Having established the relationship between the seeds and the gland, conventional X-ray imaging may then be used to locate the seeds and therefore infer the position of the prostate. The requirement to implant the gold seeds is clearly invasive, often leading to an initial inflammation of the prostate, and demands skilled resources to locate the seeds before therapy can begin.

Another problem that can occur is instability of the position of the prostate gland during treatment. The prostate lies adjacent the wall of the rectum, and any gas build-up in the rectum or colon during treatment can displace the position of the prostate.

As a result of these difficulties, it is difficult to accurately judge the position of the prostate gland, and common practice is to irradiate a slightly larger target area to ensure that the tumour receives an adequate dose of radiation. This can increase the risk of inadvertent damage to the adjacent rectal wall.

The present invention attempts a solution to some of these problems.

International Patent Application WO 03/088833 (The Johns Hopkins University) describes apparatus for insertion of a medical device such as a needle into a mammalian body during a medical imaging process. The apparatus has an elongate cylinder, sized to fit in the anal canal of a user, the interior of the cylinder having a heterogeneous internal structure arranged to define landmarks detectable by X-ray or MRI.

International Patent Application WO 03/070294 (Mayo Foundation for Medical Education and Research) describes a trans-rectal ultrasound probe holder and a method for stabilizing the prostate while imaging the prostate region or performing other diagnostic or therapeutic procedures.

International Patent Application WO 2005/046792 (Kim, Jin-il) describes a medical device for treating prostate diseases by using near-infrared light emitting diodes and, particularly, a vibrator, constructed to be inserted into the rectum.

US Patent 5,404,881 (Technomed International) describes a transrectal probe of flexible polymer material for positioning an instrument for detection or therapeutic treatment of an organ, such as the prostate.

US Patent 5,797,950 (Jiro Takashima) describes an apparatus for releasing congested prostate fluid that massages the prostate upon contraction of the anal sphincter muscle.

### Summary of the Invention

Accordingly, the invention provides a location and stabilization device, to assist a clinician in locating the position of an internal organ of a patient, comprising: an elongate cylinder, sized to fit in the anal canal of a user; the interior of said cylinder having a heterogeneous internal structure arranged to define landmarks detectable by X-ray or magnetic resonance imaging; a handle at one end of the cylinder; and characterised in that: said cylinder has a bend located at or towards the axial mid-region of the cylinder; and the device has a constriction between said cylinder and said handle; thereby anchoring the device, in use, at a repeatable depth in the anal canal of a user.

Provision of such a constriction renders the device "self-locating": as the anal sphincter contracts after the device is inserted, it grips the device at the location of the constriction thus anchoring it at the correct - and repeatable - depth. It is preferable that the sides of the constriction are angled at between approximately 45 and 60 degrees for secure location, and ease of removal of the device. The corners of the constriction have smooth transitions, again for ease of removal of the device and comfort of the patient.

The device is particularly suitable for stabilizing and locating the position of the prostate gland, although, with small changes in geometry, would also be suitable for locating and stabilizing other organs or structures lying close to the anal canal such as the bladder or other pelvic organs. For clarity, the invention will be described in relation to its use with respect to the prostate. The cylinder may have various cross-sectional profiles, not necessarily circular, nor even symmetric, but for the comfort of the patient, and ease of insertion, a circular or oval cross-section is to be preferred.

Preferably, the cylinder has a bend in the range from 20 to 60 degrees, said bend being located at or towards the axial mid-region of the cylinder.

The bend is located in the cylinder to match the profile of the internal anatomy of the anal canal and rectum when the device is in position. When in position, the bend aligns such that it is adjacent to the position of the prostate gland, the bend effectively "pointing to" the prostate. For adult human use, the bend is ideally located so it lies approximately between 30 and 50 mm internal of the anal sphincter when the device is in position. The diameter of the cylinder is preferably of such as dimension as to lie flush against the internal wall of the rectum and anus, and preferably to slightly distend it. In this way, the wall of the colon is slightly stretched and consequently moved partially away from the prostate - i.e. from the site of radiotherapy treatment. This has the effect of reducing inadvertent damage to the rectum and anal wall. An appropriate diameter (for adult human use) would be in the range of 10 to 25mm, with a diameter of approximately 15mm being particularly appropriate. The heterogeneous internal structure may comprise differing materials that show different opacities to X-rays or that show contrast in magnetic resonance imaging, or other imaging system to be used. For example, metal inserts within a plastics body may be used to provide contrast for X-ray imaging, and an aqueous gel within a plastics body would provide contrast for MRI. A particularly preferred aspect where the heterogeneity is provided by air-filled voids is described below.

Preferably, the angle of the bend is approximately 40 degrees. This corresponds to an "average" anatomy, and makes one design of the device usable with a range of patient anatomies.

Alternatively, the cylinder is narrowed at or towards the axial mid-region of the cylinder. Such a feature (as with the bend, described above) also serves to locate the device adjacent an internal structure of interest - such as the prostate gland.

Also, in any aspect of the invention it is preferable that the cylinder is tapered. Such a tapered construction allows the device to be inserted more easily into the anus (narrow end first) without trauma to the sphincter.

Also, in any aspect of the invention it is preferable that the device further comprises a handle at one end of the cylinder; the handle being essentially flat and having a mid-plane lying in or about the longitudinal mid-plane of the bent cylinder. Whilst the provision of a handle at one end makes the device easier to remove, particular advantages spring from an essentially flat construction: Once inserted into the anus, the handle lies between the buttocks of a patient, and is effectively gripped there. This provides a resistance to any rotational movement of the device and acts to ensure that the device is located in the same position each time it is used.

Also, in any aspect of the invention it is preferable that the device has an internal channel providing gaseous communication from one end to the other. It is known that a build-up of gas is likely to occur in the colon and rectum and, if this occurs during the treatment cycle, it can distend the colon and rectal wall, leading to a displacement of the prostate. With the device in place, build-up of flatus could also displace the device itself. The provision of a gaseous communication channel from one end to the other allows the gas to escape to atmosphere.

Also, in any aspect of the invention it is preferable that the landmarks include a linear feature aligned to substantially bisect the angle defined by the bent cylinder. Provision of such features gives a clear indication to the attending clinician of the position of the prostate in relation to the device.

Also, in any aspect of the invention it is preferable that the heterogeneous internal structure comprises air-filled voids. Air-filled voids provide good contrast on X-Ray and MRI images, and the use of air as the second material in the internal structure facilitates manufacture and reduces overall cost of the device. In a particularly preferred embodiment, the device may be manufactured from plastics by injection moulding. High impact polystyrene is a particularly appropriate material as it has appropriate mechanical properties, and has been found to be mucous membrane acceptable.

More preferably, the device is of separable two-part construction, so allowing access to the internal voids. With this feature, contrast markers may be inserted into the voids. Where X-rays are to be used for imaging the device, contrast markers in the form of strips of metal may be inserted into the air-filled voids, to further facilitate imaging of the landmarks. Where other imaging techniques are to be used, alternative materials for contrast markers will be evident to the skilled addressee. In particularly advantageous embodiments, the two parts are connectable by hollow studs adapted to receive contrast markers. In a related advantageous development, the internal (air-filled) voids define pockets adapted to receive contrast markers.

The scope of the invention includes, therefore, such a device further comprising one or more contrast markers. It is particularly advantageous that at least one contrast marker is in the form of a ring. The benefit of a ring-shaped marker is that its image gives information on the orientation of the marker. For example, if a circular ring is used, when imaged "face-on" it will appear as a circle; if the device is tilted with respect to the image plane, the image of the marker will become progressively more oval in shape. Similarly, if the ring is viewed "end-on" the image will be of a line; if the device is tilted with respect to the image plane, the image will become progressively more oval.

Also included within the scope of the invention is a device as claimed herein for use in a method of locating the prostate gland of a patient on multiple occasions comprising the steps of: inserting a prostate gland location and stabilization device as described above into the anus of a patient; using an imaging system to obtain an image of the device's landmarks and the prostate gland; determining and recording the spatial relationship between the landmarks and the prostate gland; and on a subsequent occasion: inserting a prostate gland location and stabilization device as described above into the anus of a patient; using an imaging system to obtain an image of the device's landmarks; and using said previously recorded spatial relationship to determine the location of the prostate gland.

### Brief Description of the Drawings

The invention will be described with reference to the accompanying drawings, in which:
Figure 1 is a cross-sectional view of a device for location and stabilisation of the prostate gland;
Figure 2 is a schematic diagram of a device showing a linear landmark;
Figure 3 illustrates images of a ring-shaped contrast marker;
Figure 4 illustrates corresponding two halves of a 2-part separable construction of a device;
Figure 5 illustrates the detail of a connecting stud, adapted to receive a contrast marker;
Figure 6 and figure 7 illustrate the positioning of contrast markers within the body of a device;
Figure 8 illustrates an alternative design of handle for a device;
Figure 9 is a schematic diagram illustrating the device in use;
Figure 10 is an end view of the device, in use.

### Description of the Preferred Embodiments

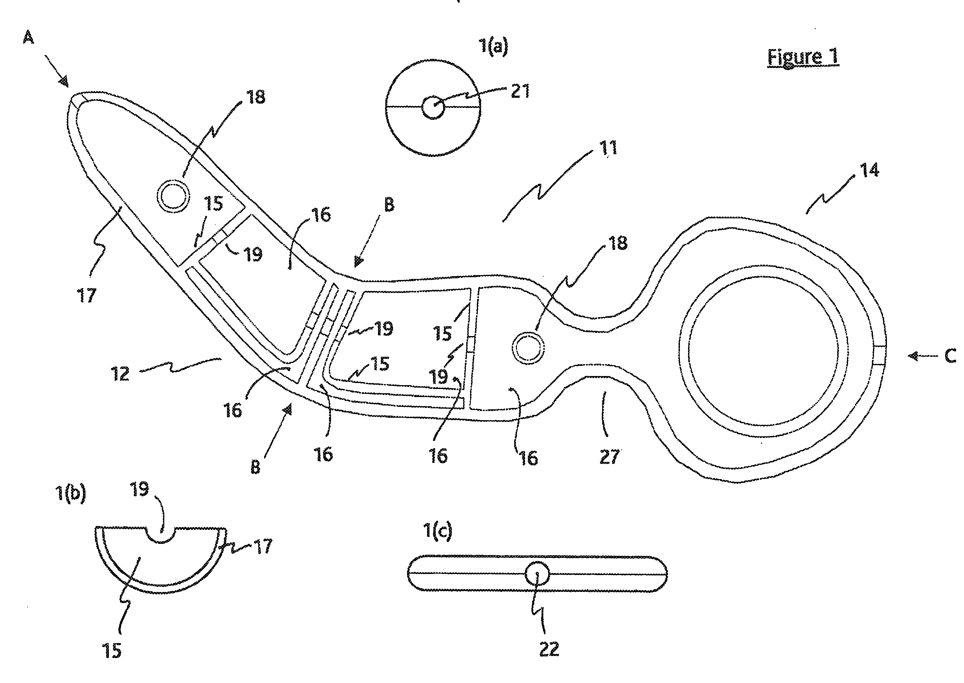

Figure 1 illustrates a cross-section through a prostate gland location and stabilisation device, generally indicated by 11, according to the present invention. The device comprises an elongate cylinder 12 having a bend 13 located towards the axial mid-region of the cylinder 12. The device has a handle 14 at one end of the cylinder 12 enabling the device to be conveniently fitted in, and subsequently removed from, the anus of a patient. The heterogeneous internal structure is formed by the presence of a series of internal ribs 15 defining air-filled voids 16 between them, and between the ribs 15 and the outer shell 17 of the device 11.

The illustration shows one half of a 2-part construction, and two connecting studs 18 are also illustrated. The studs 18 are hollow to enable contrast markers to be located within the studs, if required. This feature is discussed in more detail below.

The device has an internal channel providing gaseous communication from one end to the other. In this embodiment, this is achieved by providing a series of cut-outs 19 in the ribs 15, so connecting air-filled void 16 to form a channel from one end to the other. Figure 1(a) is an end view of device along the line of arrow A and shows a hole 21 formed in the outer shell 17 of the device 11 providing gaseous communication between the interior and exterior of the device 11. In particularly preferred embodiments, more than one hole is provided at this end on the device, preferably located at or adjacent the tip of the device; the provision of multiple holes ensures that the gas communication channel remains open, and not blocked by faecal material. (Figure 1 (b) is a cross-section along the line B-B of one half of the 2-part device and illustrating the form of the cut-out 19 in the rib 15. Figure 1 (c) is an end view of the handle 14 along the line of arrow C. The view shows a second hole 22 formed in the outer skin 17 of the device, again providing gaseous communication between the interior and exterior of the device 11.

The device 11 has a constriction 27 located between the handle 14 and the cylinder 12. As discussed above, the constriction is so shaped and sized as to be gripped by the anal sphincter muscle of the patient when the device is in use. This provides a secure and, more importantly, reproducible means of locating the device in relation to the prostate gland.

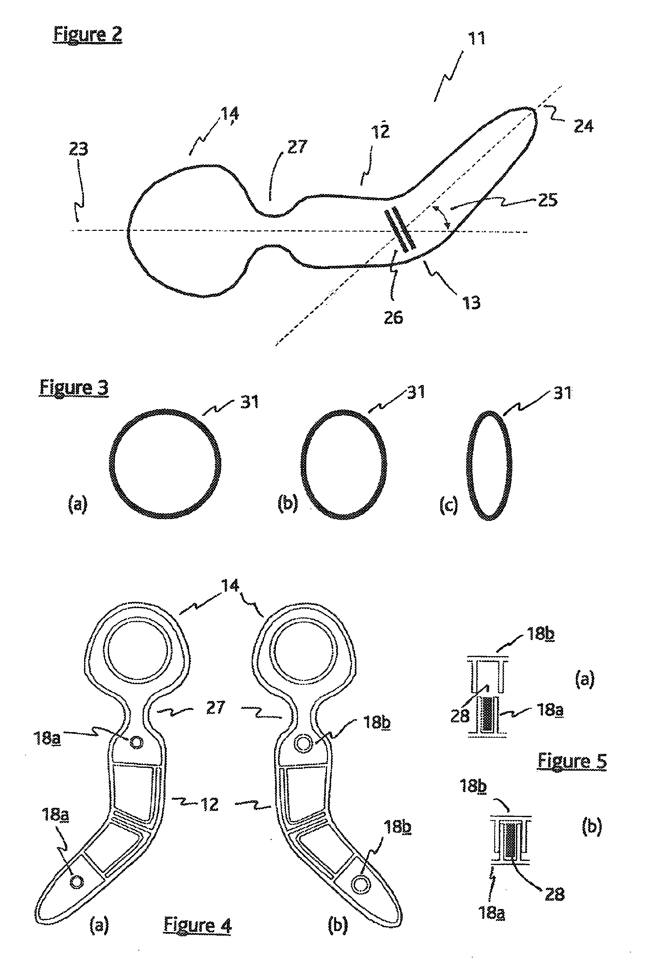

Figure 2 is a schematic diagram of a device 11 having a tapered cylinder 12 with a bend 13 towards its axial mid-region, and a handle 14. The bend 13 in the device defines two axes 23 and 24 defining an angle of bend 25. Together, the two non-parallel axes 23 and 24 define a longitudinal mid-plane of the bent cylinder. Internal landmarks 26 in this device are of linear form, and are aligned to substantially bisect the obtuse angle defined by the bent cylinder.

Figure 4 illustrates two corresponding halves of a device formed as a 2-part construction. Two pairs of interacting studs 18a and 18b are illustrated, to clip the two halves of the device together. Figure 5 illustrates a cross-section through one pair of lugs 18 initially in the unconnected configuration, figure 5 (a), and subsequently when clipped together, figure 5 (b). It can be seen at the lugs 18 are hollow, enabling a contrast marker 28 to be located within the stud.

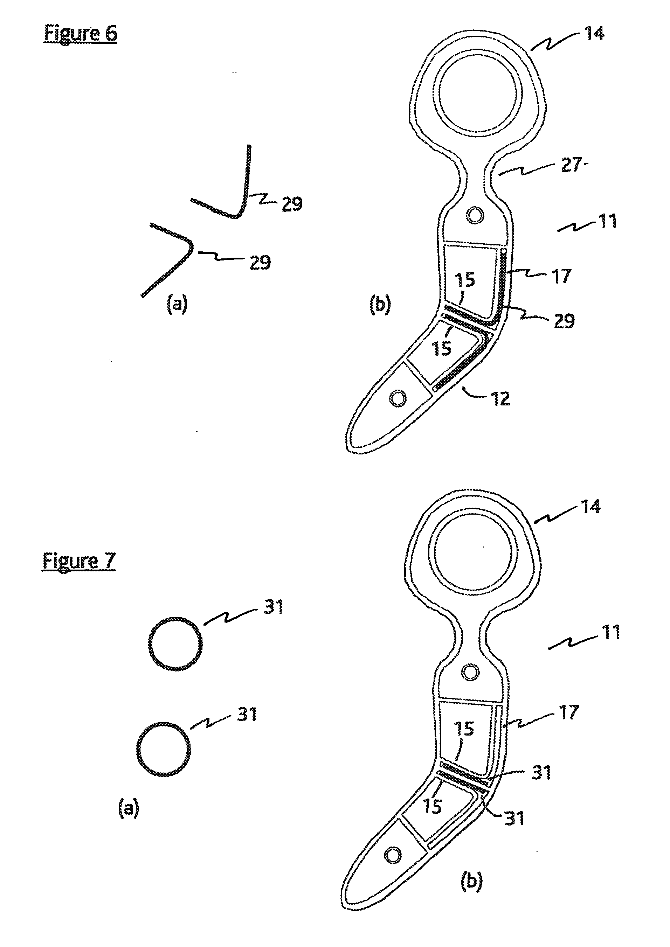

Figure 6 illustrates the use of additional contrast markers 29 in the device 11. Figure 6 (a) shows, in the plan view, a pair of contrast markers 29 formed from a strip of metal. Figure 6 (b) illustrates the positioning of the contrast markers 26 in an air-filled void between ribs 15 and the outer shell 17 of the device. In this instance, it can be seen that the contrast markers 29 define an internal landmark, of substantially linear form, and bisecting the obtuse angle defined by the bent cylinder.

Figure 7 illustrates the use of contrast markers 31 each in the form of a ring. Again, the markers 31 may be formed of a suitable material to give a high contrast on the imaging system, e.g. maybe formed of metal for x-ray imaging. In this instance, the ring-shaped contrast markers 31 are inserted into the interior of the device, and located in pockets defined by adjacent ribs 15. Again, they define a linear feature, bisecting the obtuse angle formed by the bent cylinder. The particular advantage of using ring-shaped contrast markers 31 is illustrated in figure 3. Figure 3 (a) illustrates an image of the marker 31 when viewed "end on", i.e. parallel to the image plane. Figures 3 (b) and 3 (c) illustrate the resultant image as the device is twisted with respect to an image plane. The changing shape allows a radiographer or radiologist to determine, therefore, whether the device is aligned as required.

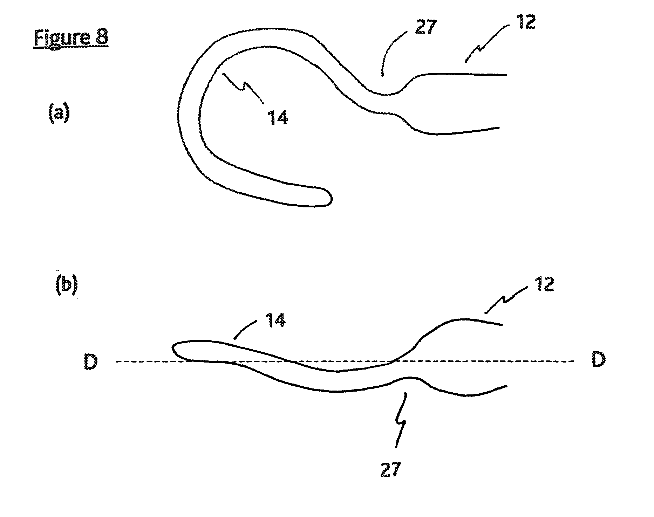

Figure 8 illustrates an alternative design of handle 14 connected to the cylinder 12 (shown in part). Figure 8 (a) is a plan view and figure 8 (b) is a side view. It can be seen from figure 8 (b) that the handle 14 is essentially flat, and defines a mid-plane indicated by the dashed line D-D.

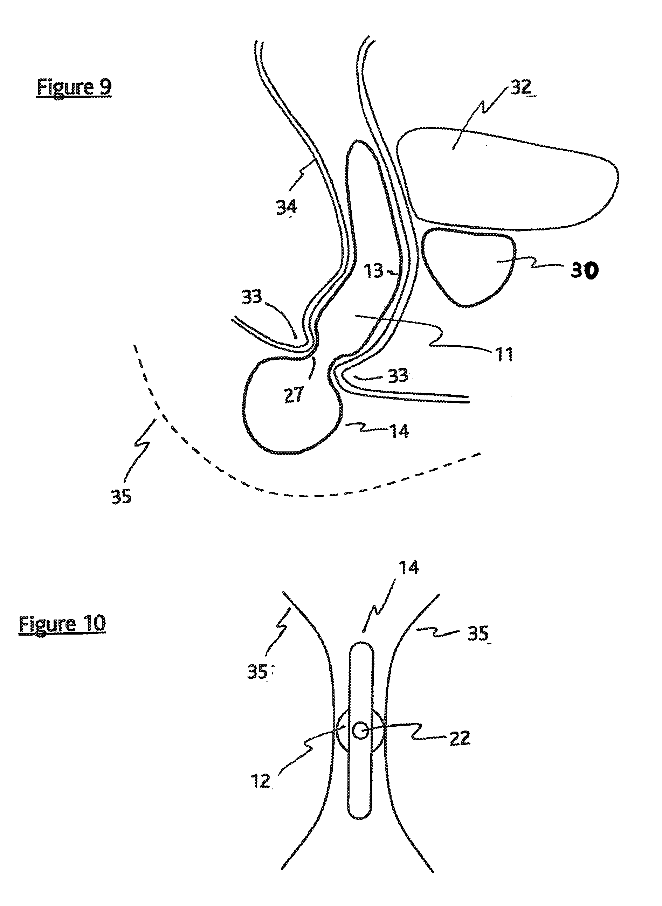

Figure 9 is a schematic cross-section of a device 11 *in situ* in the anal canal of a patient. The diagram illustrates the position of the prostate gland 30, the bladder 32, the location of the anal sphincter 33, the wall of the colon 34 and the outline of the patient's buttocks 35. It can be seen that the device follows the contour of the wall of the colon 34, with the bend 13 of the cylinder lying adjacent the prostate gland 30. As discussed above, the diameter of the device is such that it sits against the colon wall 34, and preferably distends it slightly, to stretch the colon wall tissue away from the site of treatment in the prostate gland 30. The diagram illustrates how the constriction 27 in the device 11 is gripped by the anal sphincter 33 to positively locate the device in the desired position. The diagram also illustrates how the handle 14 of the device sits between the buttocks of a patient in use.

Figure 10 is an end view of the handle 14 of the device *in situ* in the anus of a patient, illustrating how the essentially flat form of the handle 14 is gripped by the buttocks 35 of the patient. In use, the buttocks 35 would close to more closely grip the handle 14 than is illustrated in figure 10, for clarity. The resilient nature of the buttocks 35 mean that the handle 14 does not have to be absolutely flat, in the literal sense, but essentially flat, defining a mid-plane as illustrated in figure 8.

## Claims

1. A location and stabilization device [11], to assist a clinician in locating the position of an internal organ of a patient, comprising:
an elongate cylinder [12], sized to fit in the anal canal of a user;
the interior of said cylinder [12] having a heterogeneous internal structure [15, 16] arranged to define landmarks detectable by X-ray or magnetic resonance imaging;
a handle [14] at one end of the cylinder [12], and **characterised in that**:
said cylinder [12] has a bend [13] located at or towards the axial mid-region of the cylinder; and the device has constriction [27] between said cylinder [12] and said handle [14], thereby anchoring the device, in use, at a repeatable depth in the anal canal of a user.

2. A device according to Claim 1 wherein the cylinder [12] has a bend [13] in the range from 20 to 60 degrees.

3. A device according to Claim 2 wherein the angle of the bend [13] is approximately 40 degrees.

4. A device according to any of Claims 1 to 3 wherein the cylinder [12] is tapered.

5. A device according to any preceding Claim wherein said handle [14] is essentially flat and having a mid-plane lying in or about the longitudinal mid-plane of the bent cylinder.

6. A device according to any preceding Claim wherein the device has an internal channel [19] providing gaseous communication from one end to the other.

7. A device according to any preceding Claim wherein the landmarks [26] include a linear feature aligned to substantially bisect the angle defined by the bent cylinder.

8. A device according to any preceding Claim wherein the heterogeneous internal structure comprises air-filled voids [16].

9. A device according to Claim 8 wherein the device is of separable two-part construction, so allowing access to the internal voids [16].

10. A device according to Claim 9 wherein the two parts are connectable by hollow studs [18a, 18b] adapted to receive contrast markers [28].

11. A device according to either of Claims 9 and 10 wherein the internal voids [16] define pockets adapted to receive contrast markers [28]

12. A device according to either of Claims 10 and 11 further comprising one or more contrast markers [26,28,29,31]

13. A device according to Claim 12 wherein at least one contrast marker is in the form of a ring [31].

14. A device according to any preceding claim for use in a method of locating the prostate gland [30] of a patient on multiple occasions comprising the steps of:
inserting said device into the anus of a patient;
using an imaging system to obtain an image of the device's landmarks [26,28,29,31] and the prostate gland [30];
determining and recording the spatial relationship between the landmarks [26,28,29,31] and the prostate gland [30] ;
and on a subsequent occasion:
inserting a device according to any preceding claim into the anus of a patient;
using an imaging system to obtain an image of the device's landmarks [26,28,29,31] and using said previously recorded spatial relationship to determine the location of the prostate gland [30].

## Patentansprüche

1. Lokalisierungs- und Stabilisierungsgerät (11), das einen Kliniker bei der Lokalisierung der Position eines inneren Organs eines Patienten unterstützt, umfassend:
einen langen Zylinder (12), dessen Größe das Einführen in den Analkanal eines Anwenders ermöglicht;
wobei das Innere des Zylinders (12) eine heterogene interne Struktur (15, 16) aufweist, die so ausgelegt ist, dass sie Orientierungspunkte definiert, die durch Röntgenstrahlen- oder Magnetresonanz-Bildgebung erkennbar sind;
einen Handgriff (14) an einem Ende des Zylinders (12); und **dadurch gekennzeichnet, dass**:
der Zylinder (12) eine Biegung (13) aufweist, die sich in der axialen Mittelregion des Zylinders oder in deren Nähe befindet;
und dass das Gerät eine Verengung (27) zwischen dem Zylinder (12) und dem Handgriff (14) aufweist; wodurch das Gerät bei seiner Verwendung in einer wiederholbaren Tiefe im Analkanal eines Anwenders verankert werden kann.

2. Gerät gemäß Anspruch 1, wobei der Zylinder (12) eine Biegung (13) aufweist, die in der Größenordnung zwischen 20 und 60 Grad liegt.

3. Gerät gemäß Anspruch 2, wobei der Winkel der Biegung (13) ungefähr 40 Grad beträgt.

4. Gerät gemäß einem der Ansprüche 1 bis 3, wobei der Zylinder (12) spitz zulaufend ist.

5. Gerät gemäß einem der vorherigen Ansprüche, wobei der Handgriff (14) im Wesentlichen flach ist und eine Mittelebene aufweist, die in der längs verlaufenden Mittelebene des gebogenen Zylinders oder in deren Nähe liegt.

6. Gerät gemäß einem der vorherigen Ansprüche, wobei das Gerät einen internen Kanal (19) aufweist, der die Gaskommunikation von einem Ende zum anderen ermöglicht.

7. Gerät gemäß einem der vorherigen Ansprüche, wobei die Orientierungspunkte (26) ein lineares Merkmal einschließen, das so ausgerichtet ist, dass es im Wesentlichen den Winkel in zwei Hälften teilt, der durch den gebogenen Zylinder definiert wird.

8. Gerät gemäß einem der vorherigen Ansprüche, wobei die heterogene interne Struktur mit Luft gefüllte Hohlräume (16) umfasst.

9. Gerät gemäß Anspruch 8, wobei das Gerät eine trennbare Konstruktion aus zwei Teilen aufweist, sodass der Zugang zu den internen Hohlräumen (16) ermöglicht wird.

10. Gerät gemäß Anspruch 9, wobei die beiden Teile durch Hohlstifte (18a, 18b) verbunden werden können, die dazu ausgelegt sind, Kontrastmarker (28) aufzunehmen.

11. Gerät gemäß einem der Ansprüche 9 und 10, wobei die internen Hohlräume (16) Taschen definieren, die dazu ausgelegt sind, Kontrastmarker (28) aufzunehmen.

12. Gerät gemäß einem der Ansprüche 10 und 11, des Weiteren umfassend einen oder mehrere Kontrastmarker (26, 28, 29, 31).

13. Gerät gemäß Anspruch 12, wobei mindestens ein Kontrastmarker die Form eines Rings (31) hat.

14. Gerät gemäß einem der vorherigen Ansprüche zur Verwendung bei einem Verfahren zur Lokalisierung der Prostata (30) eines Patienten bei mehreren Gelegenheiten, wobei das Verfahren die folgenden Schritte umfasst:
das Einführen des Geräts in den Anus eines Patienten;
das Verwenden eines Bildgebungssystems zur Gewinnung eines Bilds von den Orientierungspunkten (26, 28, 29, 31) des Geräts und der Prostata (30);
das Ermitteln und Aufzeichnen der räumlichen Beziehung zwischen den Orientierungspunkten (26, 28, 29, 31) und der Prostata (30);
und bei einer nachfolgenden Gelegenheit:
das Einführen eines Geräts gemäß einem der vorherigen Ansprüche in den Anus eines Patienten;
das Verwenden eines Bildgebungssystems zur Gewinnung eines Bilds von den Orientierungspunkten (26, 28, 29, 31) des Geräts; und
das Verwenden der zuvor aufgezeichneten räumlichen Beziehung, um die Position der Prostata zu ermitteln.

## Revendications

1. Un dispositif de localisation et de stabilisation [11], destiné à aider un clinicien à localiserla position d'un organe interned'un patient, comprenant :
un cylindre allongé [12], dimensionné de façon à entrer dansle canal anald'un utilisateur,
l'intérieur dudit cylindre [12] possédant une structure interne hétérogène [15, 16] agencée de façon à définirdes points de repèredétectables parrayons X ou imagerie à résonance magnétique,
une poignée [14] à une extrémitédu cylindre [12], et **caractérisé en ce que** :
ledit cylindre [12] possèdeune courbure [13] située sur ou
versla zone axiale médianedu cylindre,
et le dispositif possède un étranglement [27] entreledit cylindre [12] et ladite poignée [14], ancrant ainsile dispositif, en utilisation, à une profondeur répétabledans le canal anald'un utilisateur.

2. Un dispositifselon la Revendication 1 où le cylindre [12] possède une courbure [13] dans la plage allant de 20 à 60 degrés.

3. Un dispositifselon la Revendication 2 où l'angle dela courbure [13] est approximativement de 40 degrés.

4. Un dispositif selon l'une quelconque des Revendications 1 à 3 où le cylindre [12] est conique.

5. Un dispositif selon l'une quelconque des Revendications précédentes où ladite poignée [14] est essentiellement plate et possède un plan médiansitué dans ou autour du plan médian longitudinaldu cylindre courbé.

6. Un dispositifselon l'une quelconque des Revendications précédentes oùle dispositif possède un canal interne [19] fournissantune communication gazeused'une extrémité à l'autre.

7. Un dispositifselon l'une quelconque des Revendications précédentes où les points de repère [26] incluent une caractéristique linéairealignée de façon à sensiblement bissecterl'angledéfini parle cylindre courbé.

8. Un dispositifselon l'une quelconque des Revendications précédentes où la structure interne hétérogènecomprenddes vides remplis d'air [16].

9. Un dispositifselon la Revendication 8 où le dispositifest d'uneconstruction séparable en deux parties, permettant ainsid'accéder auxvides internes [16].

10. Un dispositifselon la Revendication 9 où les deux partiespeuvent être raccordées par des goujons creux [18a, 18b] adaptés de façon à recevoirdes marqueurs de contraste [28].

11. Un dispositifselon la Revendication 9 ou 10 où les vides internes [16] définissent des pochesadaptées de façon à recevoirdes marqueurs de contraste [28].

12. Un dispositifselon la Revendication 10 ou 11 comprenant en outreun ou plusieurs marqueurs de contraste [26, 28, 29, 31].

13. Un dispositifselon la Revendication 12 où au moins unmarqueur de contrasteest de la forme d'un anneau [31].

14. Un dispositifselon l'une quelconque des Revendications précédentes destiné à une utilisation dans un procédé de localisation de la prostate [30] d'un patientà plusieurs occasions comprenant les opérations suivantes :
l'insertion dudit dispositifdans l'anusd'un patient,
l'utilisation d'unsystème d'imageriepour obtenir une imagedes points de repère du dispositif [26, 28, 29, 31] et de laprostate[30],
la détermination et l'enregistrement dela relation spatialeentreles points de repère [26, 28, 29, 31] et la prostate [30],
et à une occasion ultérieure :
l'insertion d'undispositifselon l'une quelconque des Revendications précédentesdans l'anusd'un patient,
l'utilisation d'unsystème d'imagerie pour obtenir une imagedes points de repère du dispositif [26, 28, 29, 31],et l'utilisation de ladite relation spatialeenregistrée antérieurementafin de déterminer la localisation dela prostate.
